# EUROPÄISCHE PATENTSCHRIFT

(11) **EP 0 871 400 B1**
(45) Veröffentlichungstag und Bekanntmachung des Hinweises auf die Patenterteilung: **01.03.2000**
(21) Anmeldenummer: 96943915.7
(22) Anmeldetag: 11.12.1996
(51) Int. Cl.: A61B 5/12

(54) **ERMITTLUNG VON DATEN ÜBER DAS HÖRVERMÖGEN**
OBTAINING DATA ON HEARING CAPACITY
DETERMINATION DE DONNEES SUR LA CAPACITE AUDITIVE

(30) Priorität: 29.12.1995 DE 19549165
(43) Veröffentlichungstag der Anmeldung: 21.10.1998
(73) Patentinhaber: Plinkert, Peter K., 72127 Kusterdingen (DE); Schnitzler, Hans-Ulrich, 72076 Tübingen (DE); Zenner, Hans-Peter, 72070 Tübingen (DE); Heitmann, Jürgen, 72074 Tübingen (DE); Waldmann, Bernd, 72074 Tübingen (DE)
(72) Erfinder: HEITMANN, Jürgen, D-72074 Tübingen (DE); WALDMANN, Bernd, D-72074 Tübingen (DE)
(74) Vertreter: Patentanwälte Ruff, Beier, Schöndorf und Mütschele
(86) Internationale Anmeldenummer: EP9605527
(87) Internationale Veröffentlichungsnummer: WO9724065

(56) Entgegenhaltungen:
- HEARING RESEARCH, Bd. 86, Nr. 1-2, Juni 1995, DIE NIEDERLANDE, Seiten 47-62, XP000672455 KETTEMBEIL, MANLEY, SIEGL: "Distortion-product otoacoustic emissions and their anaesthesia sensitivity in the European Starling and the chicken"

## Beschreibung

Die Erfindung betrifft ein Verfahren zur Ermittlung von Daten über das Hörvermögen durch Messung von Distorsionsprodukten otoakustischer Emissionen, d. h. durch Messung von sogenannten DPOAE.

Beim Menschen als Repräsentant von Wirbeltieren und insbesondere von Säugern ist das Hörvermögen nach gängiger Ansicht mit aktiven Verstärkungsprozessen im Innenohr verknüpft, die die Empfindlichkeit und Frequenzauflösung des Ohres insgesamt verbessern. Als Epiphänomen derartiger Prozesse werden die sogenannten otoakustischen Emissionen angesehen. Dies sind aus dem Ohr emittierte Schallsignale, die spontan auftreten oder durch äußere Stimulation hervorgerufen, d. h. evoziert werden können. Die Stimulation kann dabei beispielsweise akustisch oder elektrisch erfolgen.

Die grundsätzliche Bedeutung evozierter otoakustischer Emissionen für die Ermittlung von Daten über das Hörvermögen wurde bereits vor längerer Zeit erkannt. So ist in der EP-B1-15258 von Kemp beschrieben, daß als Reaktion auf ein Schallereignis Schallemissionen im äußeren Gehörgang gemessen werden können, die mit dem Zustand im Ohr in Zusammenhang stehen. Dabei wurden die otoakustischen Emissionen durch Kemp mit Hilfe einer akustischen Sonde, bestehend aus einem hochempfindlichen Miniaturmikrophon und einem Schallsender gemessen.

Weiter ist bekannt, Distorsionsprodukte otoakustischer Emissionen zu bestimmen, die durch eine bitonale Stimulation des Hörorgans, beim Menschen der Cochlea mit zwei Reintönen, den sogenannten Primärtönen entstehen. Dabei treten Schallemissionen bei den Frequenzen nf₁ ± mf₂ mit ganzen Zahlen n und m auf. Beim Menschen ist insbesondere das sogenannte kubische Distorsionsprodukt 2f₁ - f₂ gut meßbar. Bestimmungen derartiger DPOAE zeigten jedoch keine gute Korrelation zwischen der Amplitude des Distorsionsprodukts und konventionell gemessenen Werten für die Hörschwelle.

Durch Hinzufügen eines dritten Stimulustones als sogenannter Störton oder Suppressor kann die DPOAE-Amplitude beeinflußt werden. Aus solchen Messungen wurde im Stand der Technik bisher der Schluß gezogen, daß die DPOAE-Amplitude durch einen Suppressionston im Bereich der beiden Primärtöne beeinflußt werden kann, ein Suppressionston in der Nähe der Frequenz des Distorsionsprodukts jedoch keinen Einfluß auf die DPOAE-Amplitude hat. In diesem Zusammenhang wird als Stand der Technik auf die Veröffentlichtung von Plinkert, Harris und Probst in HNO (1993) 41: 339 - 344 und auf die Veröffentlichung von kettembeil, Manley und Siegl in Hearing Research (1995) Bd. 86, Nr. 1-2, Die Niederlande, Seiten 47-62 verwiesen.

Alle bisherigen Bemühungen, auf dem Gebiet der Messung von otoakustischen Emissionen und Distorsionsprodukten otoakustischer Emissionen haben letzten Endes das Ziel, ein Verfahren zur objektiven Messung des Hörvermögens zur Verfügung zu stellen. Ein solches Verfahren ist deshalb von großer Bedeutung, weil dabei beispielsweise keine (subjektiven) Angaben einer menschlichen Versuchsperson als Reaktion auf den Stimulus mehr erforderlich sind und Messungen an verschiedensten Hörorganen miteinander vergleichbar werden können.

Die Erfindung stellt sich deshalb die Aufgabe, ein solches objektives Verfahren zur Messung des Hörvermögens zu entwickeln bzw. ein Verfahren, das diesen Anforderungen weitgehend entspricht. Dabei sollen die genannten Nachteile vermieden werden. Die Entwicklung eines solchen Verfahrens soll ebenso in die Bereitstellung einer entsprechenden Vorrichtung und einer dafür geeigneten Sonde münden.

Diese Aufgabe wird gelöst durch ein Verfahren mit den Merkmalen des Anspruchs 1. Bevorzugte Ausführungsformen sind in den abhängigen Ansprüchen 2 bis 12 beschrieben. Der Wortlaut sämtlicher Ansprüche wird durch Bezugnahme zum Inhalt dieser Beschreibung gemacht.

Bei dem erfindungsgemäßen Verfahren werden als Stimuli zwei Primärtöne mit den Frequenzen f₁ und f₂ > f₁ und den Schalldruckpegeln L₁ und L₂ sowie mindestens ein Schallereignis mit Anteilen der Frequenz f₃ und dem Schalldruckpegel L₃ auf das Hörorgan aufgebracht und ein Distorsionsprodukt definierter Frequenz bestimmt. Dabei besitzt die Frequenz f₃ einen Wert, der in der Nähe der Frequenz des Distorsionsproduktes liegt.

Durch dieses Verfahren wird unter anderem erreicht, daß die Abhängigkeit der Emissionsamplitude des Distorsionsproduktes von der Frequenz der Stimuli vergleichsweise einfach bestimmt werden kann. Die Erstellung von Meßdaten der Emissionsamplitude des Distorsionsproduktes in Abhängigkeit von der Frequenz der Stimuli, ein sogenanntes DP-Gramm kann mit wenigen Meßpunkten hinreichend genau abgetastet werden. Der bei routinemäßigen Messungen auftretende Nachteil, daß die Feinstruktur bei einer Beschränkung auf wenige Meßfrequenzen nicht abgetastet werden kann, wird durch die Anwendung des erfindungsgemäßen Verfahrens überwunden.

Der in Anspruch 1 gewählte Ausdruck "in der Nähe der Frequenz des Distorsionsproduktes" bedeutet in erster Linie, daß die Frequenz f₃ nicht im Bereich der Frequenz f₂ oder im Bereich zwischen den Frequenzen f₁ und f₂ liegt. Vorzugsweise wird die Frequenz f₃ möglichst nahe an die Frequenz des Distorsionsproduktes angenähert, wobei diese Annäherung durch die apparativen Gegebenheiten begrenzt sein kann. Beispielsweise erfolgt bei einer gemäß der Erfindung verwendbaren Apparatur die Annäherung bis auf Frequenzen von 10 oder 12,5 Hz, da diese Frequenzauflösung aus dem verwendeten Datentransformationssystem resultiert. Üblicherweise ist die Frequenz f₃ nicht mit der Frequenz des Distorsionsproduktes identisch, da eine Auftrennung der beiden Frequenzen im gemessenen Signal erforderlich ist.

Bei bevorzugten Ausführungsformen des erfindungsgemäßen Verfahrens liegt die Frequenz f₃ knapp oberhalb des Wertes für die Frequenz des Distorsionsproduktes.

Als Schallereignis, das als Störton oder Suppressor verwendet wird, wird vorzugsweise ein dritter (Rein-) Ton mit der Frequenz f₃ und dem Schalldruckpegel L₃ aufgebracht. Dies führt dann zu einer Stimulation des Hörorgans mit drei Reintönen der Frequenzen f₁, f₂ und f₃ und den Schalldruckpegeln L₁, L₂ und L₃.

In Weiterbildung ist der Schalldruckpegel L₃ des Schallereignisses, insbesondere des dritten Tons kleiner als die Schalldruckpegel L₁ oder L₂ mindestens eines Primärtons. Grundsätzlich besteht der Zusammenhang, daß der Schalldruckpegel L₃ beispielsweise bei einem dritten Ton mit der Frequenz f₃ ausreichend groß sein muß, um die Vorteile zu erreichen, wie sie bereits beschrieben wurden und noch beschrieben werden. Üblicherweise kann der Schalldruckpegel L₃ um so kleiner gewählt werden, je näher die Frequenz f₃ an der Frequenz des Distorsionsprodukts liegt. Wie erwähnt läßt sich das Verfahren besonders gut durchführen, wenn f₃ etwas oberhalb der Frequenz des Distorsionsproduktes liegt. Je weiter entfernt die Frequenz f₃ von der Frequenz des Distorsionsproduktes gewählt wird, desto größer muß im Normalfall der Schalldruckpegel L₃ gewählt werden.

Bei weiteren bevorzugten Ausführungsformen weicht der Schalldruckpegel L₃ des Schallereignisses bzw. des dritten Tons nicht wesentlich vom Schalldruckpegel L₂ des Primärtons mit der Frequenz f₂ ab. Dabei kann der Schalldruckpegel L₃ etwas kleiner als der Schalldruckpegel L₂ gewählt werden. Vorzugsweise ist der Schalldruckpegel L₃ gleich dem Schalldruckpegel L₂.

Zweckmäßige Abweichungen des Schalldruckpegels L₃ vom Schalldruckpegel L₂ liegen im Bereich zwischen -15 dB bis +15 dB. Innerhalb dieses Bereichs sind insbesondere Abweichungen zwischen -10 dB und +10 dB bevorzugt.

Grundsätzlich können bei der Erfindung alle möglichen Distorsionsprodukte bestimmt werden. Dabei sind manche Distorsionsprodukte besonders gut bestimmbar, beispielsweise aufgrund der Intensitäten der entsprechenden Schallemissionen. Beim Menschen kann insbesondere das sogenannte kubische Distorsionsprodukt 2f₁ - f₂ bestimmt werden.

Die Durchführung des erfindungsgemäßen Verfahrens erfolgt vorzugsweise im sogenannten Audiobereich, d. h. dem "normalen" Hörbereich. Innerhalb dieses Bereichs wird das Verfahren insbesondere bei Frequenzen zwischen 500 Hz und 16 kHz durchgeführt, d. h. die Frequenz der Stimuli wird innerhalb des genannten Frequenzbereiches variiert.

Die Abgabe der Stimuli kann grundsätzlich im Laufe der Meßdauer auf unterschiedliche Weise erfolgen. Vorzugsweise werden die Stimuli, insbesondere die Primärtöne auf das Hörorgan kontinuierlich aufgebracht. Wird eine intermittierende Abgabe der Stimuli auf das Hörorgan gewählt, wird insbesondere das als Suppressor dienende Schallereignis intermittierend eingesetzt.

Bei der Erfindung kann zusätzlich die aus den Eigenschaften der Meßapparatur resultierende Erscheinung, daß das Analysezeitfenster, d. h. der Zeitraum innerhalb dem die Messung erfolgt, begrenzt ist, in vorteilhafter Weise ausgenutzt werden. Wird nämlich die Frequenz der Stimuli als ganzzahliges Vielfaches der sich aus dem Kehrwert des Analysezeitfensters (in Sekunden) ergebenden Grundfrequenz (in Hertz) gewählt, entfallen auf die Länge des Zeitfensters der nachfolgenden Analyse eine ganze Anzahl von Wellen aller drei Stimuli. Dies bedingt eine wesentliche Vereinfachung der Auswertung. Dementsprechend wird bei der Erfindung vorzugsweise eine definierte Kopplung von drei auf das Hörorgan aufgebrachten Tönen untereinander bewirkt, wobei diese Kopplung letzten Endes durch eine Einrichtung zur digitalen Datenverarbeitung mit zugehöriger Digital-Analog-Wandlung hervorgerufen ist. Dabei ist der Takt von beispielsweise drei zur Erzeugung von drei Tönen vorgesehener D/A-Wandler entsprechend synchronisiert.

Weiter kann das erfindungsgemäße Verfahren so ausgestaltet sein, daß bei der Ermittlung der Meßdaten Frequenz und Schalldruckpegel eines Primärtons, insbesondere f₂ und L₂ des zweiten Primärtons, vorgegeben werden. Dann werden aufgrund vorgegebener, beispielsweise aus Versuchen stammender Kriterien vorteilhafte Frequenz- und Schalldruckpegelwerte für den anderen Primärton, insbesondere den ersten Primärton mit f₁ und L₁ sowie den Suppressor, insbesondere den dritten Ton mit f₃ = f_{DP} ± X berechnet und anschließend die Messung durchgeführt.

Bei dem erfindungsgemäßen Verfahren kann weiter zusätzlich eine Kalibrierung erfolgen, daß beispielsweise die spezifischen Gegebenheiten des jeweiligen Gehörgangs eines menschlichen Probanden individuell berücksichtigt werden. Zu diesem Zweck wird vor Beginn der eigentlichen Messung ein Stimulus über alle interessierenden Frequenzen (weißes Rauschen) aufgegeben und aufgenommen. Dann kann bei der späteren Messung für jede einzelne Frequenz eine Korrektur vorgenommen werden, die die individuelle Gestaltung des jeweiligen Gehörgangs berücksichtigt. Werden zur Erzeugung der Stimuli beispielsweise elektroakustische Wandler verwendet, so kann bei der Erfindung für jede Frequenz ein individueller Spannungswert ermittelt werden, der den gewünschten Schalldruckpegel auf das Hörorgan erzeugt. Demgegenüber werden beim Stand der Technik üblicherweise konstante Spannungssignale verwendet, unabhängig davon, ob diese individuell tatsächlich zu dem gewünschten Schalldruckpegel führen.

Weiter kann bei der Erfindung zweckmäßig eine zeitliche Mittelung der Meßdaten für das innerhalb von Zeitfenstern bestimmte Distorsionsprodukt vorgenommen werden. Dies ist in der Erscheinung begründet, daß das eigentliche Meßsignal bei einer einzelnen Messung gegenüber dem Hintergrundrauschen nicht oder nur schwer erkennbar ist, wobei bei einer Vielzahl von Messungen mit Mittelung das Rauschen ausgemittelt wird. Bei einer bevorzugten Ausführungsform der Erfindung können die bei der Mittelung erhaltenen Werte laufend mit dem Umgebungs- bzw. Hintergrundrauschen abgeglichen und bewertet werden. Dadurch wird erreicht, daß die Mittelung jederzeit nach Erreichen eines gewünschten Signal-Rausch-Verhältnisses, d.h. eines bestimmten Abstands zwischen DPOAE-Signal und dem darunterliegenden Hintergrundrauschen, abgebrochen werden kann und keine feste Anzahl von Zyklen (Zeitfenstern) durchlaufen werden muß. Damit kann die Meßdauer insgesamt verkürzt werden.

Bei dem erwähnten Hörorgan handelt es sich vorzugsweise um ein Hörorgan von Wirbeltieren, insbesondere von Säugetieren. Bei Säugetieren und beim Menschen kann es sich bei dem Hörorgan insbesondere um die Cochlea handeln.

Der Verlauf von bei der Erfindung erhaltenen Meßkurven, bei denen die Feinstruktur zum Teil ausgelöscht wird und sogenannte "flache" DP-Gramme werden im Zusammenhang mit den Figuren noch beschrieben. Eine Erklärung der Vorteile des erfindungsgemäßen Verfahrens kann darin liegen, daß die Feinstruktur eines DP-Grammes durch die Interferenz zweier räumlich getrennter Generationsmechanismen bzw. Generatoren für die Emission bei der Frequenz des Distorsionsproduktes zurückzuführen ist. Man kann davon ausgehen, daß das Distorsionsprodukt beim Menschen in dem Bereich auf der Basilarmembran der Cochlea entsteht, in dem sich die beiden Primärtöne überlappen. Dabei entstehen durch die nichtlineare Arbeitsweise des cochlären Verstärkers im Überlappungsbereich der Frequenzen f₁ und f₂, und dabei in der Gegend von f₂, Verzerrungen, die sich in Form von Distorsionsprodukten äußern. Diese Verzerrungstöne breiten sich von dem Ort ihrer Entstehung aus und erreichen so das Mittelohr und produzieren über das Trommelfell einen im Gehörgang meßbaren Schalldruck. Weiter breiten sich die Verzerrungstöne auch in die entgegengesetzte Richtung aus und führen an dem Ort, der 2f1-f2 repräsentiert, zu einer resonanzbedingten Auslenkung der Basilarmembran. Diese Schwingung wird durch den aktiven cochleären Prozeß verstärkt und breitet sich ebenfalls retrograd zum Mittelohr aus (Stimulusfrequenz-Otoakustische Emission (SFOAE). Dementsprechend treten am Trommelfell und im Gehörgang zwei Sinustöne der gleichen Frequenz, nämlich der Frequenz des Distorsionsproduktes, in Erscheinung, die jedoch von zwei räumlich getrennten Emissionsorten, nämlich der genannten Stelle in der Gegend von f₂ und von der Stelle mit f_{dp} stammen. Diese beiden Signale interferieren in Abhängigkeit von der relativen Phasenlage. Auf diese in Abhängigkeit von der Frequenz unterschiedliche Phasenlage kann die Feinstruktur mit ihrer Abfolge von Minima und Maxima (Rippling) zurückgeführt werden.

Bei Aufbringen eines konkurrierenden Suppressors gemäß der Erfindung kann nun die Stimulusfrequenzotoakustische Emission (SFOAE) bei der Frequenz f_{dp} separat ausgeschaltet werden. Ein Suppressor in Nachbarschaft zu der Frequenz des Distorsionsproduktes zieht die Energie des Verstärkers auf seine Frequenz. Damit lassen sich auch die geschilderten Abhängigkeiten der Suppression von Frequenz und Schalldruckpegel des Suppressors erklären.

Die Erfindung läßt sich dementsprechend auch darstellen durch ein Verfahren zur Ermittlung von Daten über das Hörvermögen durch Messung von Distorsionsprodukten otoakustischer Emissionen (DPOAE), bei dem zwei Primärtöne mit den Frequenzen f₁ und f₂ > f₁ und den Schalldruckpegeln L₁ und L₂ als Stimuli auf das Hörorgan aufgebracht werden und mindestens ein Distorsionsprodukt definierter Frequenz bestimmt wird, wobei bei diesem Verfahren bei Vorliegen von mindestens zwei Generatoren bzw. Generationsmechanismen für das Distorsionsprodukt mindestens einer dieser Generatoren bzw. Generationsmechanismen supprimiert wird. Eine derartige Suppression kann in völlig beliebiger Weise erfolgen und ist grundsätzlich nicht auf das Aufbringen von Schallereignissen beschränkt. Werden Schallereignisse zur Suppression verwendet, weist das Verfahren mindestens eines der oben beschriebenen Merkmale auf. Dementsprechend wird auf die bisherige Beschreibung verwiesen und Bezug genommen.

Eine Vorrichtung zur Durchführung des erfindungsgemäßen Verfahrens umfaßt erste Mittel zur Erzeugung von mindestens drei Stimuli, insbesondere akustischer Stimuli, zweite Mittel zur Detektion otoakustischer Emissionen, insbesondere damit verbundener Schallemissionen, und dritte Mittel zur Steuerung und Auswertung, insbesondere für die Erzeugung von Stimuli sowie die Erfassung und Aufarbeitung von Meßdaten.

Die ersten Mittel können insbesondere sogenannte elektroakustische Wandler aufweisen, wie sie aus dem Stand der Technik bekannt sind. Grundsätzlich kann dabei ein elektroakustischer Wandler zur Erzeugung von zwei oder mehreren Stimuli vorgesehen sein. So kann beispielsweise ein elektroakustischer Wandler einen Primärton und den Suppressionsstimulus (Suppressionston) erzeugen. Es ist jedoch bevorzugt, wenn für jeden Stimulus ein separater Wandler, d. h. für zwei Primärtöne und einen Suppressionston drei elektroakustische Wandler vorgesehen sind.

In Weiterbildung weisen die zweiten Mittel mindestens ein Mikrophon zur Detektion der evozierten Schallereignisse auf. Dabei können mehrere Mikrophone, beispielsweise vier vorgesehen sein, aus denen Meßwerte gemittelt werden.

Die dritten Mittel weisen vorzugsweise eine Einrichtung zur digitalen Datenverarbeitung mit zugehöriger D/A-Wandlung und A/D-Wandlung auf. Dabei sind zur Erzeugung von zwei Primärtönen und einem Stimulus, insbesondere Suppressionston drei D/A-Wandler vorgesehen. In entsprechender Weise ist zur Detektion der evozierten Schallereignisse ein A/D-Wandler vorhanden.

Bezüglich der weiteren Ausgestaltung der Vorrichtung wird auf die Beschreibung der entsprechenden Stellen beim erfindungsgemäßen Verfahren verwiesen, auf die hier ausdrücklich Bezug genommen wird.

Eine Vorrichtung zur Durchführung des erfindungsgemäßen Verfahrens umfaßt eine Sonde oder einen Meßkopf für die Ermittlung von Daten über das Hörvermögen, der bzw. dem mindestens drei Einrichtungen für die Emission von Schallstimuli, insbesondere von Tönen definierter Frequenz und mindestens eine Einrichtung zur Detektion von Schallemissionen zugeordnet ist. Bei den Einrichtungen für die Emission von Schallstimuli handelt es sich vorzugsweise um elektroakustische Wandler und bei den Detektionseinrichtungen vorzugsweise um Mikrophone.

In Weiterbildung sind bei der Sonde sämtliche Einrichtungen, d. h. vorzugsweise drei elektroakustische Wandler und ein Mikrophon integriert. Dadurch wird eine kompakte Bauweise mit möglichst naher Anordnung der Einrichtungen am Meßort erreicht.

Bei bevorzugten Ausführungsformen ist die Sonde mindestens teilweise in den äußeren Gehörgang eines Säugers, insbesondere des Menschen einführbar, womit ebenfalls die Meßgenauigkeit erhöht wird. Zusätzlich kann ein Dichtelement zum weitgehenden Ausschluß störender äußerer Schallereignisse (Umgebungsschall) vorgesehen sein.

Die beschriebenen Merkmale und weitere Merkmale der Erfindung ergeben sich aus der nachfolgenden Beschreibung von bevorzugten Ausführungsformen in Verbindung mit den Unteransprüchen, den Zeichnungen und dem Beispiel. Hierbei können die einzelnen Merkmale jeweils für sich oder zu mehreren in Kombination miteinander verwirklicht sein.

In den Zeichnungen zeigen:
- Figur 1: die Darstellung des Spektrums zur Erläuterung der Erfindung,
- Figur 2: ein Blockschaltbild einer Ausführungsform der Vorrichtung zur Durchführung der Erfindung
- Figur 3: eine schematische Darstellung einer Sonde zur Durchführung der Erfindung
- Figur 4: einen Querschnitt durch die Spitze einer Sonde gemäß Figur 3,
- Figur 5: ein sogenanntes DP-Gramm zur Erläuterung der Erfindung, und
- Figur 6: die Darstellung von Suppressions-Wachstums-Kurven zur Erläuterung der Erfindung.

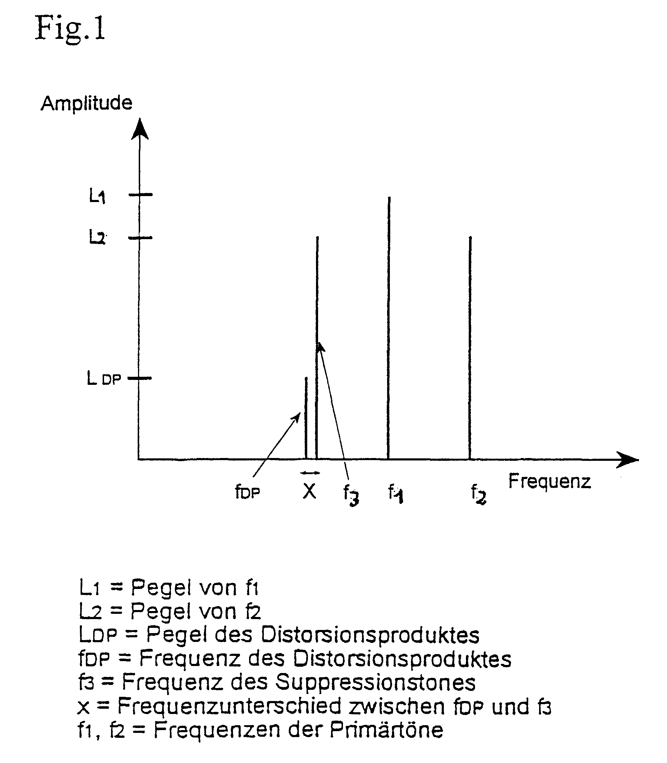

Aus Figur 1 sind gleichzeitig die Grundlagen und wesentliche Merkmale der Erfindung ersichtlich. Die verwendeten Symbole und Abkürzungen sind in der Legende zu Figur 1 erläutert.

Durch die schmalen Balken bei den Frequenzen f₁ und f₂ sind die beiden Primärtöne mit den Schalldruckpegeln L₁ und L₂ symbolisiert. Bei der dargestellten Ausführung besitzt der zweite Primärton eine höhere Frequenz als der erste Primärton bei geringerem Schalldruckpegel L₂. Das Distorsionsprodukt mit dem Schalldruckpegel L_{DP} ist durch den schmalen Balken bei der Frequenz f_{DP} symbolisiert. Knapp oberhalb bei höheren Frequenzen ist ein Suppressionston mit der Frequenz f₃ dargestellt, wobei der Frequenzunterschied zwischen Frequenz des Distorsionsproduktes und Frequenz des Suppressionstons x beträgt. Der Schalldruckpegel L₃ des Suppressionstones ist gleich dem Schalldruckpegel L₂ des zweiten Primärtones gewählt.

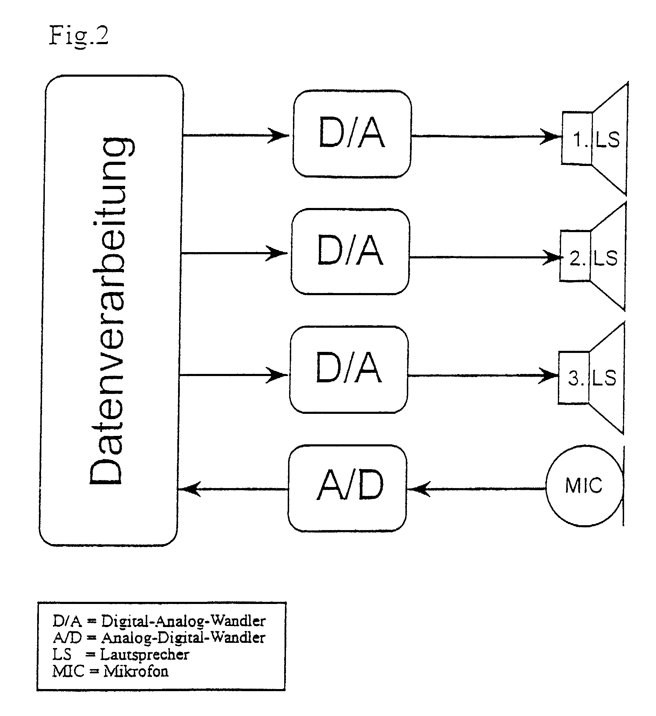

Das Blockschaltbild gemäß Figur 2 zeigt schematisch wesentliche Teile einer Vorrichtung, wobei die Abkürzungen und Symbole wiederum in einer Legende erläutert sind. Gemäß Figur 2 besitzt die Vorrichtung drei Digital-Analog-Wandler und einen Analog-Digital-Wandler, die einer Auswerte- und Steuerungseinrichtung zugeordnet sind, die in Figur 2 ganz allgemein als Datenverarbeitung bezeichnet ist. Mit Hilfe dieser Datenverarbeitung werden die Wandler gesteuert, d. h. letzten Endes die auf das Hörorgan aufgebrachten Stimuli erzeugt und die evozierten Schallereignisse ausgewertet. Die Digital-Analog-Wandler sind jeweils mit einem Lautsprecher verbunden, die den eigentlichen Schallstimulus der insgesamt gebildeten elektroakustischen Wandler hervorbringen. Der Analog-Digital-Wandler steht mit einem Mikrophon in Verbindung, mit dessen Hilfe das evozierte Schallereignis detektiert wird.

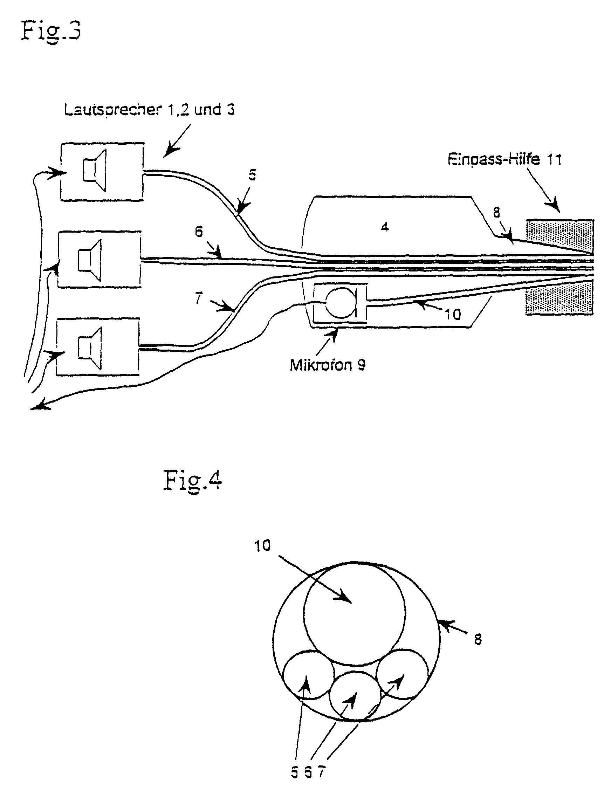

Figur 3 zeigt die schematische Ansicht einer Sonde, der drei Lautsprecher 1, 2 und 3 sowie ein Mikrophon 9 zugeordnet sind. Die drei Lautsprecher stehen mit dem eigentlichen Sondenkörper über Schlauchleitungen 5, 6 und 7 in Verbindung, die durch diesen hindurch zu einer Sondenspitze 8 geführt sind. Im vorliegenden Fall ist ein Mikrophon 9 in den Sondenkörper 4 integriert und ebenfalls über eine dünne Schlauchleitung 10 in die Sondenspitze 8 geführt. Wie bereits erwähnt, könnten zusätzlich auch die Lautsprecher 1, 2 und 3 in den Sondenkörper 4 integriert sein.

Am Außenumfang der in Richtung auf das Hörorgan spitz, insbesondere konisch zulaufenden Sondenspitze 8 ist eine Einpaßhilfe 11 angeordnet, die beim Einsetzen der Sonde in den äußeren Gehörgang zur besseren Einführung und anschließenden Festlegung dient. Die Einpaßhilfe 11 ist vorzugsweise aus einem weichen und elastischen Material gefertigt.

Figur 4 zeigt eine Querschnittsansicht der Sondenspitze 8, wobei die jeweiligen Enden der dünnen Schlauchleitungen 5, 6, 7 und 10 dargestellt sind. Diese Schlauchleitungen sind in Richtung auf das Hörorgan offen, so daß zwischen den Lautsprechern 1, 2 und 3 sowie dem Mikrophon 9 und dem Trommelfell, das in den Figuren nicht dargestellt ist, ein durchgehendes Luftpolster vorhanden ist. Gemäß Figur 4 besitzt die Schlauchleitung 10 für das Mikrophon 9 einen größeren Querschnitt als die drei Schlauchleitungen 5, 6 und 7 der Lautsprecher 1, 2 und 3.

### Beispiel

Am Ohr einer Versuchsperson werden DPOAE-Messungen durchgeführt, wobei die in den Figuren dargestellte Vorrichtung bzw. Sonde zum Einsatz kommen kann. Dabei werden Primärtöne mit Schalldruckpegeln von L₁ = 65 dB SPL und L₂ = 55 dB SPL bei verschiedenen Frequenzen mit und ohne Einsatz eines Suppressionstones (Suppressor) über Außenohr und Mittelohr auf die Cochlea aufgebracht.

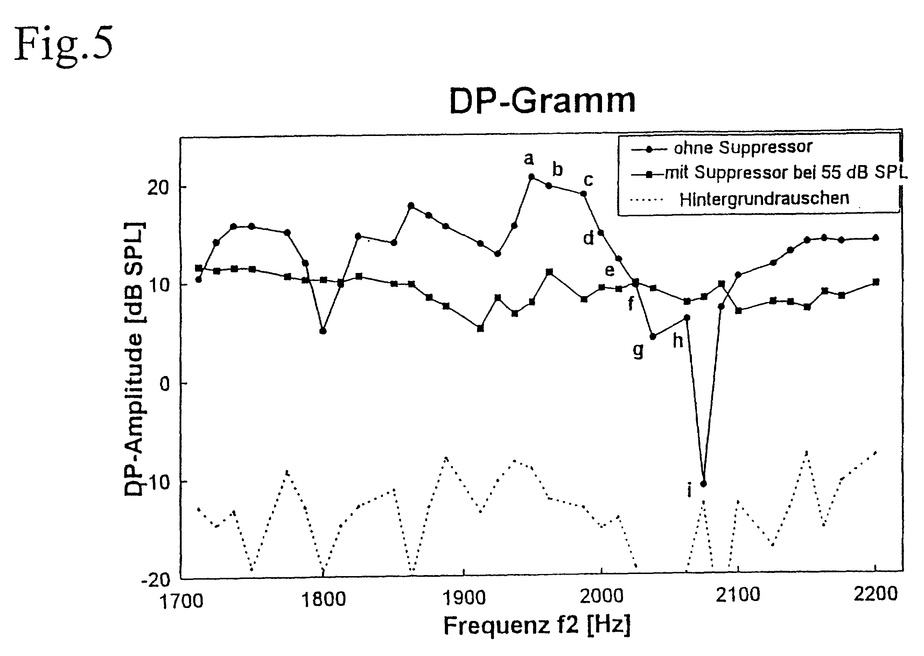

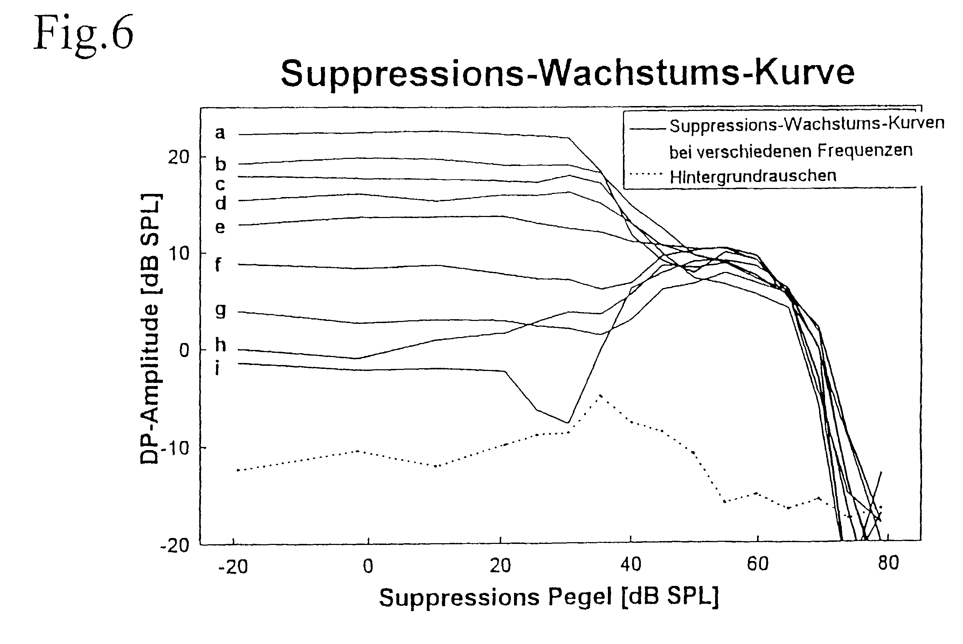

Zur Erläuterung der im Beispiel erhaltenen Meßergebnisse dienen die beiden Figuren 5 und 6.

In Figur 5 ist die Amplitude des gemessenen Distorsionsproduktes 2f₁ - f₂ in Abhängigkeit von der Frequenz des zweiten Primärtons dargestellt. Dabei symbolisieren die runden Meßpunkte Messungen ohne Aufbringen eines Suppressionstones, während die quadratischen Meßpunkte Messungen symbolisieren, bei denen ein Suppressionston mit der Frequenz 2f₁ - f₂ + 12,5 Hz und einer Lautstärke von 55 dB SPL auf das Hörorgan eingestrahlt wurde.

Figur 5 zeigt, daß die Meßkurve ohne Suppressor, die eine große Unschärfe in der Größenordnung der Amplitudensprünge zwischen benachbarten Minima und Maxima aufweist, durch das erfindungsgemäße Verfahren in eine vergleichsweise flache Kurve überführt werden kann, welche kaum mehr ausgeprägte Amplitudensprünge aufweist. Zur Erklärung dieses Effekts kann die in der Beschreibungseinleitung bereits dargestellte Theorie herangezogen werden.

Figur 6 zeigt den Suppressionseffekt in Abhängigkeit von der Lautstärke des Suppressionstons. Dieses Suppressionsverhalten unterscheidet sich erheblich in Abhängigkeit von der Frequenz der Stimulustöne. In Bereichen eines Maximums in der Feinstruktur verringert sich die DP-Amplitude, in Bereichen eines Minimums verstärkt sich die Emissionsamplitude jedoch. Dieses Phänomen zeigt sich sowohl im Glättungseffekt gemäß Figur 5 als auch in den Wachstumskurven gemäß Figur 6, in der die in Figur 5 mit a bis i bezeichneten Meßpunkte in Abhängigkeit von der Lautstärke des Suppressionstons aufgenommen sind.

Abnahme und Anwachsen der Emissionsamplitude mit Variation des Suppressionspegels läßt sich gemäß der geschilderten Theorie über die Interferenz zweier gleicher Signale mit gegenläufiger bzw. gleichsinniger Phase erklären.

Bei Durchführung des erfindungsgemäßen Verfahrens wird also der hemmende bzw. verstärkende Einfluß eines zweiten Generators für das Distorsionsprodukt entfernt und das gemessene Signal ist ausschließlich auf den anderen Generator zurückzuführen. Damit kann dieser (Haupt-) Generator, der wie im vorliegenden Fall Rückschlüsse über den Zustand des Innenohrs ermöglicht, zuverlässig bestimmt werden. Das übriggebliebene Signal ist nur noch mit dem Zustand des Innenohrs an der Stelle des verbleibenden Generators, d. h. beim (2f₁ - f₂)-Distorsionsprodukt mit der Stelle bei der Frequenz f₂ assoziiert und kein Mischsignal mehr.

Schließlich zeigt Figur 6, daß im dargestellten Fall bei Suppressionspegeln ab ca. 35 dB SPL ein erster Suppressionseffekt einsetzt, der bei den Kurven zu g, h, i zu einer Verstärkung der Emission führt. Der Suppressionseffekt ist bis zu einem Schalldruckpegel des Suppressionstons von ca. 65 dB SPL erkennbar. Erst oberhalb dieses Suppressionspegels beeinflußt der Suppressionston den anderen ("Hauptgenerator") bei f₂, dessen Bestimmung ohne störende Einflüsse erwünscht ist. Im Bereich zwischen einem Suppressionspegel von 45 dB SPL und 65 dB SPL ändert sich im vorliegenden Fall an der DP-Amplitude so gut wie nichts, und zwar unabhängig davon, bei welcher Frequenz gemessen wird. Der dargestellte Effekt ist zwar bei verschiedenen Menschen unterschiedlich stark ausgeprägt, jedoch bei allen nachweisbar.

## Patentansprüche

1. Verfahren zur Ermittlung von Daten über das Hörvermögen durch Messung von Distorsionsprodukten otoakustischer Emissionen (DPOAE), bei dem als Stimuli zwei Primärtöne mit den Frequenzen f₁ und f₂ > f₁ und den Schalldruckpegeln L₁ und L₂ sowie mindestens ein Schallereignis mit Anteilen der Frequenz f₃ und dem Schalldruckpegel L₃ auf das Hörorgan aufgebracht werden und mindestens ein Distorsionsprodukt definierter Frequenz gemessen wird, dadurch gekennzeichnet, daß f₃ einen Wert besitzt, der in der Nähe der Frequenz des Distorsionsproduktes liegt.

2. Verfahren nach Anspruch 1, dadurch gekennzeichnet, daß als Schallereignis ein dritter Ton mit der Frequenz f₃ und dem Schalldruckpegel L₃ aufgebracht wird.

3. Verfahren nach Anspruch 1 oder 2, dadurch gekennzeichnet, daß der Schalldruckpegel L₃ des Schallereignisses, insbesondere des dritten Tons kleiner als ein Schalldruckpegel L₁ oder L₂ mindestens eines Primärtons ist.

4. Verfahren nach einem der vorhergehenden Ansprüche, dadurch gekennzeichnet, daß der Schalldruckpegel L₃ des Schallereignisses, insbesondere des dritten Tons nicht wesentlich vom Schalldruckpegel L₂ des Primärtons mit der Frequenz f₂ abweicht, vorzugsweise gleich oder geringfügig kleiner als der Schalldruckpegel L₂ des Primärtons mit der Frequenz f₂ ist.

5. Verfahren nach einem der vorhergehenden Ansprüche, dadurch gekennzeichnet, daß der Schalldruckpegel L₃ des Schallereignisses, insbesondere des dritten Tons um -15 dB bis +15 dB, insbesondere um -10 dB bis +10 dB vom Schalldruckpegel L₂ des Primärtons mit der Frequenz f₂ abweicht.

6. Verfahren nach einem der vorhergehenden Ansprüche, dadurch gekennzeichnet, daß das sogenannte (2f₁ - f₂)-Distorsionsprodukt bestimmt wird.

7. Verfahren nach einem der vorhergehenden Ansprüche, dadurch gekennzeichnet, daß es im sogenannten Audiobereich, vorzugsweise bei Frequenzen zwischen 500 Hz und 16 kHz durchgeführt wird.

8. Verfahren nach einem der vorhergehenden Ansprüche, dadurch gekennzeichnet, daß die Abgabe der Stimuli auf das Hörorgan kontinuierlich erfolgt.

9. Verfahren nach einem der Ansprüche 1 bis 7, dadurch gekennzeichnet, daß die Abgabe der Stimuli auf das Hörorgan intermittierend erfolgt.

10. Verfahren nach einem der vorhergehenden Ansprüche, gekennzeichnet durch eine definierte Kopplung von drei auf das Hörorgan aufgebrachten Tönen untereinander, vorzugsweise mit Hilfe einer Einrichtung zur digitalen Datenverarbeitung mit zugehöriger Digital-Analog-Wandlung.

11. Verfahren nach einem der vorhergehenden Ansprüche, gekennzeichnet durch eine zeitliche Mittelung der Meßdaten für das innerhalb von Zeitfenstern bestimmte Distorsionsprodukt, wobei vorzugsweise die bei der Mittelung erhaltenen Werte laufend im Vergleich mit dem Umgebungs- bzw. Hintergrundrauschen bewertet werden.

12. Verfahren nach einem der vorhergehenden Ansprüche, dadurch gekennzeichnet, daß es sich bei dem Hörorgan um ein Hörorgan von Wirbeltieren, vorzugsweise bei Säugetieren und dem Menschen um die Cochlea handelt.

## Claims

1. A method for obtaining data on hearing capacity by measuring distortion products of otoacoustic emissions (DPOAE) in which stimuli of two primary tones having the frequencies f₁ and f₂ > f₁ and the sound pressure levels L₁ and L₂ as well as at least one sound event having components of the frequency f₃ and the sound pressure level L₃ are applied to the hearing organ and at least one distortion product of a defined frequency is determined, whereby the frequency f₃ has a value close to the frequency of the distortion product.

2. The method as set forth in claim 1, characterized in that a third tone having the frequency f₃ and the sound pressure level L₃ is applied as the sound event.

3. The method as set forth in claim 1 or 2, characterized in that the sound pressure level L₃ of the sound event, more particularly of the third tone, is smaller than a sound pressure level L₁ or L₂ of at least one primary tone.

4. The method as set forth in any of the preceding claims, characterized in that the sound pressure level L₃ of the sound event, more particularly of the third tone, does not substantially deviate from the sound pressure level L₂ of the primary tone having the frequency f₂, it being preferably equal to or slightly smaller that the sound pressure level L₂ of the primary tone having the frequency f₂.

5. The method as set forth in any of the preceding claims, characterized in that the sound pressure level L₃ of the sound event, more particularly of the third tone, deviates from the sound pressure level L₂ of the primary tone having the frequency f₂ by -15 dB to +15 dB, more particularly by -10 dB to and +10 dB.

6. The method as set forth in any of the preceding claims, characterized in that the so-called (2f₁ - f₂) distortion product is determined.

7. The method as set forth in any of the preceding claims, characterized by it being implemented in the so-called audio range, preferably at frequencies between 500 Hz and 16 kHz.

8. The method as set forth in any of the preceding claims, characterized in that emission of the stimuli to the hearing organ is made continuously.

9. The method as set forth in any of the claims 1 to 7, characterized in that emission of the stimuli to the hearing organ is made intermittently.

10. The method as set forth in any of the preceding claims, characterized by a defined intercoupling of three tones applied to the hearing organ, preferably with the aid of a means for digital data processing involving digital-to-analog conversion.

11. The method as set forth in any of the preceding claims, characterized by an averaging of the measurement data in time for the distortion product determined within time windows, the values obtained from averaging being preferably continually weighted in comparison with the ambient or background noise.

12. The method as set forth in any of the preceding claims, characterized in that the hearing organ is the hearing organ of vertebrates, preferably the cochlea of mammals and of man.

## Revendications

1. Procédé pour la détermination de données sur la capacité auditive par la mesure de produits de distorsion d'émissions oto-acoustiques, selon lequel sont appliqués en stimuli deux sons primaires avec les fréquences f₁ et f₂ > f₁ et les niveaux de pression acoustique L₁ et L₂ ainsi qu'au moins un événement sonore avec des parts de fréquence f₃ et le niveau de pression acoustique L₃ sur l'organe auditif et au moins un produit de distorsion de fréquence définie est mesuré, caractérisé en ce f₃ possède une valeur qui se rapproche de la fréquence du produit de distorsion.

2. Procédé selon la revendication 1, caractérisé en ce qu'en tant qu'événement acoustique, un troisième son avec la fréquence f₃ et le niveau de pression acoustique L₃ est appliqué.

3. Procédé selon la revendication 1 ou 2, caractérisé en ce que le niveau de pression acoustique L₃ de l'événement acoustique, en particulier du troisième son, est inférieur à un niveau de pression acoustique L₁ ou L₂ d'au moins un son primaire.

4. Procédé selon l'une des revendications précédentes, caractérisé en ce que le niveau de pression acoustique L₃ de l'événement acoustique, en particulier le troisième son, n'est pas sensiblement différent du niveau de pression acoustique L₂ du son primaire avec la fréquence L₂, est de préférence identique ou légèrement inférieur au niveau de pression acoustique L₂ du son primaire avec la fréquence f₂.

5. Procédé selon l'une des revendications précédentes, caractérisé en ce que le niveau de pression acoustique L₃ de l'événement acoustique, en particulier le troisième son diverge de -15 dB jusqu'à +15 dB, en particulier de -10 dB à +10 dB du niveau de pression acoustique L2 du son primaire avec la fréquence f₂.

6. Procédé selon l'une des revendications précédentes, caractérisé en ce que ledit produit de distorsion (2f₁ - f₂) est déterminé.

7. Procédé selon l'une des revendications précédentes, caractérisé en ce qu'il est mis en oeuvre dans ladite plage audio de préférence à des fréquences entre 500 Hz et 16 kHz.

8. Procédé selon l'une des revendications précédentes, caractérisé en ce que l'émission du stimulus s'effectue en continu sur l'organe auditif.

9. Procédé selon l'une des revendications 1 à 7, caractérisé en ce que l'émission du stimulus s'effectue par intermittence sur l'organe auditif.

10. Procédé selon l'une des revendications précédentes, caractérisé par une combinaison de trois sons appliqués sur l'organe auditif, de préférence à l'aide d'un dispositif de traitement de données numériques avec la conversion numérique analogique correspondante.

11. Procédé selon l'une des revendications précédentes, caractérisé par une communication temporelle des données de mesure pour le produit de distorsion défini à l'intérieur de fenêtres temporelles, de préférence les valeurs obtenues à l'homogénéisation étant analysées en continu en comparaison des bruits de fond et environnants.

12. Procédé selon l'une des revendications précédentes, caractérisé en ce que pour l'organe auditif, il s'agit d'un organe auditif de vertébrés, de préférence du Cochlea pour les mammifères et l'être humain.
